# EUROPÄISCHE PATENTSCHRIFT

(11) **EP 0 269 927 B1**
(45) Veröffentlichungstag und Bekanntmachung des Hinweises auf die Patenterteilung: **05.05.1993**
(21) Anmeldenummer: 87116741.7
(22) Anmeldetag: 12.11.1987
(51) Int. Cl.: A61B 6/03, H01J 35/30

(54) **Computertomograph**
Computerized tomography apparatus
Appareil de tomographie assisté par ordinateur

(30) Priorität: 25.11.1986 DE 3640195
(43) Veröffentlichungstag der Anmeldung: 08.06.1988
(73) Patentinhaber: SIEMENS AKTIENGESELLSCHAFT, 80333 München (DE)
(72) Erfinder: Klingenbeck, Klaus, Dr., D-8521 Hessdorf (DE); Oppelt, Arnulf, Dr., D-8521 Spardorf (DE)

(56) Entgegenhaltungen:
- DE-A- 2 811 464

## Beschreibung

Die Erfindung betrifft einen Computertomographen mit einer ringförmigen, das Meßfeld umschließenden Anode, auf der ein Elektronenstrahl umläuft, so daß Röntgenstrahlung zur Durchstrahlung des Meßfeldes aus verschiedenen Richtungen erzeugt wird, wobei in einer Ablenkvorrichtung für den Elektronenstrahl einem umlaufenden Magnetfeld ein periodisch variierendes Zusatzfeld überlagert wird, so daß der Elektronenstrahl die Oberfläche der Anode sowohl in azimuthaler als auch in radialer Richtung umläuft. Ein derartiger Computertomograph ist aus der DE-A- 28 11 464 bekannt.

Ein Computertomograph dieser Art weist im Gegensatz zu Ausführungen, bei denen eine Meßanordnung aus Röntgenstrahler und Strahlendetektor um das Meßfeld mechanisch herumbewegt wird, keine schweren mechanisch bewegten Teile zur Abtastung des Meßfeldes auf. Die Durchstrahlung des im Meßfeld angeordneten Patienten aus verschiedenen Richtungen kann daher sehr schnell erfolgen, so daß in äußerst kurzer Zeit ein Querschnittsbild der jeweils durchstrahlten, im Meßfeld liegenden Schicht des Patienten entsteht.

Die zur Erzeugung der Röntgenstrahlung erforderliche Elektronenstrahlung wird von einer Elektronenkanone emittiert und nach deren Verlassen elektronenoptisch, d.h. mittels elektrischer und/oder magnetischer Linsen auf die Anode fokussiert. Die erforderliche Umlaufbewegung des so erzeugten Elektronenstrahlbündels wird mittels eines entsprechend bewegten elektrischen oder magnetischen Umlenkfeldes realisiert.

Zur Erzielung einer hohen Bildschärfe im erzeugten Röntgenbild ist ein möglichst punktförmiger Fokus erforderlich. Dieser würde jedoch lokal zu Überhitzungserscheinungen an der Anode und demgemäß zu deren Zerstörung führen. Deshalb muß der Brennfleck gestreckt sein, im Idealfall als Rechteck z.B. mit dem Seitenverhältnis von 6 : 1. Stellt man die Anode unter einem kleinen Winkel - von z.B. 10° - gegen die Emissionsrichtung der Röntgenstrahlung an, hat die Projektion des Fokus wiederum die erforderliche punktförmige Ausbildung. So ist ein großer elektronischer Brennfleck mit entsprechend verminderter thermischer Belastung der Anode bei kleinem optischen Brennfleck realisierbar.

Bei einem Computertomographen der geschilderten Art mit bewegtem Elektronenstrahl führt die Aufweitung des Brennfleckes in seiner Längsrichtung dazu, daß aufwendige elektronenoptische Zusatzmaßnahmen erforderlich sind.

Der Erfindung liegt demgemäß die Aufgabe zugrunde, einen Computertomographen der eingangs genannten Art so auszubilden, daß ohne eine Aufweitung des Brennfleckes in seiner Längsrichtung bei punktförmigem optischen Brennfleck eine Überlastung der Anode verhindert ist.

Diese Aufgabe ist erfindungsgemäß dadurch gelöst, daß der Fokus auf der Anode eine ununterbrochene wellenförmige Bahn beschreibt. Dadurch vergrößert sich der Weg des Fokus auf der Anode und die lokale Erwärmung wird vermindert.

Das periodisch variierende Zusatzfeld kann einfach realisiert werden, indem man die für die Umlenkung des Elektronenstrahles erforderlichen Ströme bzw. Spannungen entsprechend periodisch variiert. Die periodische Schwankung des Fokus um einen mittleren Kreis auf dem Umfang der Anode führt jedoch zu Artefakten in der Röntgenabbildung, wenn die Wobbelfrequenz nicht deutlich größer als die Abtastfrequenz einer aufgenommenen Projektion ist. Deshalb sollten die Umlauf-, die Abtast- und die Wobbelfrequenz synchronisiert werden. Insbesondere ist es dabei vorteilhaft, die Synchronisation so auszuführen, daß in der Zeit zwischen der Aufnahme zweier zu einer Projektion gehörenden Abtastwerte eine viertel oder eine halbe Periode der Wobbelfrequenz oder ein ganzzahliges Vielfaches davon liegt. Eine weitere Verbesserung ist möglich, wenn die Synchronisation darüber hinaus so ausgeführt wird, daß zu den jeweiligen Abtastzeitpunkten der Fokus des Elektronenstrahles den mittleren Kreis auf der Anode kreuzt oder aber daß die Maxima oder Minima der radialen Auslenkung in den jeweiligen Abtastzeitpunkten liegen.

Mit diesen Maßnahmen ist nicht nur eine Verringerung der Röntgenbildartefekte möglich, sondern auch eine Reduktion der Wobbelfrequenz bis auf die halbe bzw. den vierten Teil der Abtastfrequenz einer Einzelprojektion. Dies erlaubt höhere Induktivitäten bei den Spulen für das Umlenkfeld, so daß geringere Ablenkströme möglich werden, mit entsprechend geringeren Ansprüchen an die Leistungsverstärkung des Umlenk- und Wobbelgenerators.

Durch die DE-A-28 11 464 ist ein Computertomograph der eingangs genannten Art bekannt, bei dem eine Ablenkung des Elektronenstrahles in radialer Richtung der ringförmigen Anode in der Weise erfolgt, daß der Elektronenstrahl wechselweise auf der Anode und auf einem Strahlenkollektor auftrifft. Der Strahlenkollektor dient dabei nicht zur Erzeugung von Röntgenstrahlung. Die Bahn auf der Anode ist demgemäß keine kontinuierliche wellenförmige Bahn, sondern eine unterbrochene Bahn. Die Ablenkung des Elektronenstrahles in radialer Richtung erfolgt also nicht wie bei der vorliegenden Erfindung kontinuierlich auf der Anode selbst, sondern um den Elektronenstrahl wechselweise auf den Strahlenkollektor und auf die Anode zu richten.

Die Erfindung ist nachfolgend anhand eines in der Zeichnung dargestellten Ausführungsbeispieles näher erläutert. Es zeigen:
- Fig. 1: einen Computertomographen zur Erläuterung des Erfindungsgedankens, und
- Fig. 2: die Anode und die Fokusbahn des Computertomographen gemäß Fig. 1

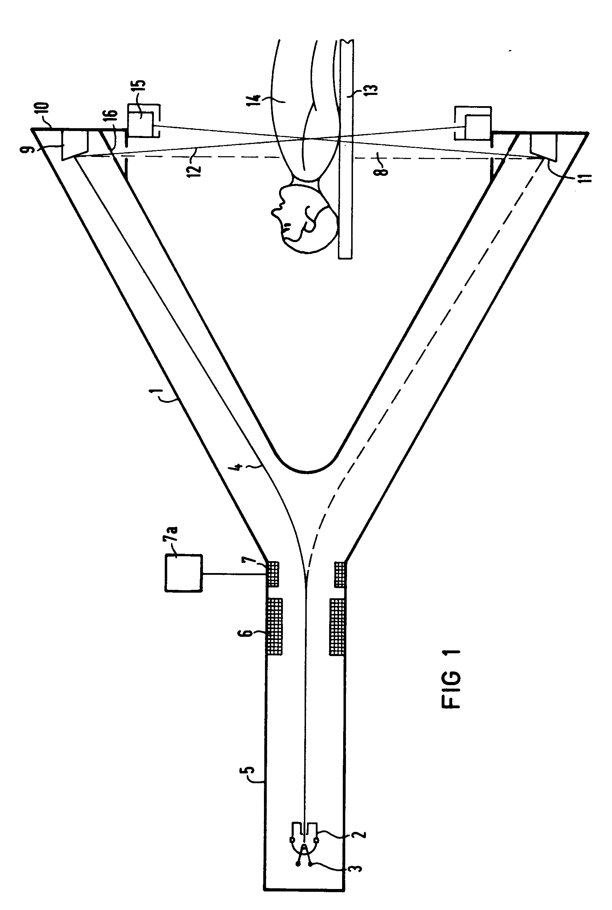

In der Fig. 1 ist ein Vakuumgefäß 1 gezeigt, das eine Elektronenkanone 2 mit einer Heizung 3 aufweist. Die Elektronenkanone 2 sendet einen gebündelten Elektronenstrahl 4 aus. Für die Bündelung ist am Hals 5 des Vakuumgefäßes 1 eine Fokussierungsspule 6 angeordnet. Am Hals 5 sitzt ferner eine Spule 7, die zur Stromsteuerung und zur Ablenkung dient. Das Vakuumgefäß 1 ist an seinem rechten Ende trichterförmig ausgebildet. Eine ringförmige, das Meßfeld 8 umschließende Anode 9 ist im rechten Ende des Vakuumgefäßes 1 an der Stirnseite 10 angeordnet. Sie besitzt eine um einen von 90° abweichenden Winkel zum Elektronenstrahl 4 geneigte Bahn 11, die durch den Elektronenstrahl 4 abgetastet wird. Das dabei entstehende Röntgenstrahlenbündel 12 wird fächerförmig eingeblendet, wobei die Fächerebene senkrecht zur Zeichenebene verläuft und tastet das Meßfeld 8 demgemäß aus verschiedenen Richtungen, d.h. unter verschiedenen Projektionen ab. Im Meßfeld 8 wird ein auf einer Liege 13 liegender Patient 14 angeordnet, von dem eine Transversalschicht vom Röntgenstrahlenbündel 12 unter verschiedenen Projektionen durchsetzt wird. Das Strahlenprofil innerhalb des Röntgenstrahlenbündels 12 wird von einem ringförmigen Strahlendetektor 15 erfaßt. Entsprechende elektrische Signale werden einem Computer zugeführt, der daraus die Schwächungswerte einer Matrix von Bildpunkten im Meßfeld 8 berechnet. Diese Schwächungswerte können bildlich wiedergegeben werden. Das Vakuumgefäß 1 besitzt ein ringförmiges Strahlenfenster 16, aus dem das Röntgenstrahlenbündel 12 austreten kann.

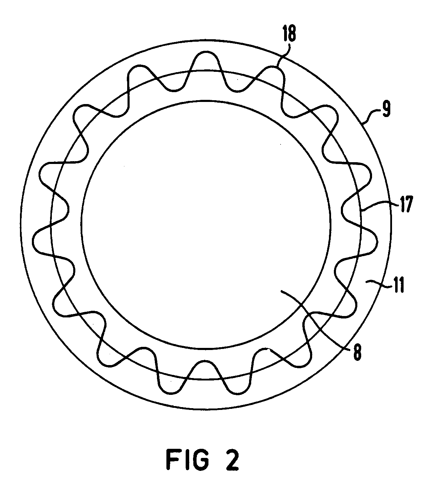

Die Fig. 2 zeigt die Bahn 11 auf der Anode 9 und den Verlauf des Brennfleckes auf dieser. Auf der Anodenbahn 11 ist ein mittlerer Kreis 17 aufgetragen, um den die Brennfleckbahn 18 periodisch pendelt, d.h. in bezug auf den die Brennfleckbahn 18 wellenförmig verläuft. Dies ist durch entsprechende Ablenkung des Elektronenstrahles 4 erreicht.

Eine Abwandlung besteht darin, daß die Anode 9 das Meßfeld 8 nicht als geschlossener Ring umschließt, sondern nur teilweise, z.B. halbkreisförmig.

Für die geeignete Wobbelung des Elektronenstrahles 4 in radialer Richtung, die der Bewegung des Elektronenstrahles 4 in Umfangsrichtung der Anode 9 überlagert ist, ist eine Steuervorrichtung 7a für die Spule 7 vorgesehen. Durch die Steuervorrichtung 7a kann eine Synchronisation der Umlauf-, der Abtast- und der Wobbelfrequenz erreicht werden. Es kann insbesondere erreicht werden, daß in der Zeit zwischen der Aufnahme zweier zu einer Projektion gehörenden Abtastwerte eine viertel oder eine halbe Periode der Wobbelfrequenz oder ein ganzzahliges Vielfaches davon liegt. Insbesondere ist erzielbar, daß zu den jeweiligen Abtastzeitpunkten der Projektionen der Fokus den Kreis 17 kreuzt oder daß in den Abtastzeitpunkten Maxima bzw. Minima der Fokusbahn 18 liegen.

## Patentansprüche

1. Computertomograph mit einer das Meßfeld (8) umschließenden Anode (9), auf der ein Elektronenstrahl (4) umläuft, so daß Röntgenstrahlung (12) zur Durchstrahlung des Meßfeldes (8) aus verschiedenen Richtungen erzeugt wird, wobei in einer Ablenkvorrichtung (7, 7a) für den Elektronenstrahl (4) einem umlaufenden Magnetfeld ein periodisch variierendes Zusatzfeld überlagert wird, so daß der Elektronenstrahl (4) die Oberfläche (11) der Anode (9) sowohl in azimuthaler als auch in radialer Richtung umläuft, **dadurch gekennzeichnet,** daß der Fokus auf der Anode (9) eine ununterbrochene, wellenförmige Bahn (18) beschreibt.

2. Computertomograph nach Anspruch 1, **dadurch gekennzeichnet,** daß die Umlauf-, die Abtast- und die Wobbelfrequenz für den Elektronenstrahl (4) synchronisiert sind.

3. Computertomograph nach Anspruch 2, **dadurch gekennzeichnet,** daß die Synchronisation so gewählt ist, daß in der Zeit zwischen der Aufnahme zweier zu einer Projektion gehörenden Abtastwerte eine viertel oder eine halbe Periode der Wobbelfrequenz oder ein ganzzahliges Vielfaches davon liegt.

4. Computertomograph nach Anspruch 3, **dadurch gekennzeichnet,** daß die Synchronisation so erfolgt, daß der Fokus zu den jeweiligen Abtastzeitpunkten der Projektionen einen mittleren Kreis (17) auf der Anode (9) kreuzt oder daß die Maxima oder Minima der radialen Fokusauslenkung in den jeweiligen Abtastzeitpunkten liegen.

## Claims

1. Computer tomography apparatus having an anode (9) which surrounds the measuring field (8) and on which an electron beam (4) circulates so that X-radiation (12) is generated for the purpose of X-raying the measuring field (8) from various directions, in which in a deflecting arrangement (7, 7a) for the electron beam (4) a periodically varying additional field is superimposed upon a circulating magnetic field so that the electron beam (4) circulates round the surface (11) of the anode (9) both in an azimuthal direction and in a radial direction, characterised in that the focus on the anode (9) describes an uninterrupted wave-shaped path (18).

2. Computer tomography apparatus according to claim 1, characterised in that the circulating frequency, the scan frequency and the sweep frequency for the electron beam (4) are synchronized.

3. Computer tomography apparatus according to claim 2, characterised in that the synchronization is selected so that a quarter or a half period of the sweep frequency or an integral multiple thereof lies within the time between receiving two scan values pertaining to a projection.

4. Computer tomography apparatus according to claim 3, characterised in that the synchronization is effected so that the focus at the respective scan times of the projections crosses a central circle (17) on the anode (9) or that the maxima or minima of the radial focus deflection lie within the respective scan times.

## Revendications

1. Tomodensitomètre comportant une anode (9), qui entoure le champ de mesure (8) et que balaye un faisceau d'électrons (4) de manière à exposer le champ de mesure à un rayonnement X (12) suivant des directions différentes, et dans lequel, dans un dispositif (7,7a) de déviation supplémentaire du faisceau d'électrons (4), un champ à variations périodiquement est superposé au champ magnétique tournant, de sorte que le faisceau d'électrons (4) balaye la surface (11) de l'anode (9) tant dans la direction azimutale que dans la direction radiale, caractérisé par le fait que le foyer décrit, sur l'anode (9), une trajectoire sinueuse ininterrompue (18).

2. Tomodensitomètre suivant la revendication 1, caractérisé par le fait que la fréquence de rotation, la fréquence d'exploration et la fréquence de vobulation du faisceau d'électrons (4) sont synchronisées.

3. Tomodensitomètre suivant la revendication 2, caractérisé par le fait que la synchronisation est choisie de telle sorte qu'il s'écoule un quart de période ou une demi-période de la fréquence de vobulation ou un multiple entier de cette valeur apparaît pendant l'intervalle de temps compris entre l'enregistrement de deux valeurs d'exploration associées à une projection.

4. Tomodensitomètre suivant la revendication 3, caractérisé par le fait que la synchronisation s'effectue de telle sorte que le foyer croise un cercle (17) sur l'anode (9) aux instants respectifs d'exploration des projections ou que les maxima ou les minima de la déviation radiale du foyer apparaissant aux instants respectifs d'exploration.
